# EUROPEAN PATENT SPECIFICATION

(11) **EP 3 850 018 B1**
(45) Date of publication and mention of the grant of the patent: **23.08.2023**
(21) Application number: 19780818.1
(22) Date of filing: 14.02.2019
(51) Int. Cl.: C08F 220/10, C08F 220/60, G02B 1/04, A61L 27/16, A61L 27/50, C08F 20/34, A61L 27/52, A61L 27/54, C08F 220/34

(54) **ANTIMICROBIAL CONTACT LENSES**
ANTIMIKROBIELLE KONTAKTLINSEN
LENTILLES DE CONTACT ANTIMICROBIENNES

(43) Date of publication of application: 21.07.2021
(73) Proprietor: KeraMed, Inc., Santa Ana, CA 92705 (US)
(72) Inventor: SHIUEY, Yichieh, Santa Ana, CA 92705 (US)
(74) Representative: Clark, Jane Anne
(86) International application number: PCT/US2019/017992
(87) International publication number: WO 2019/194903

(56) References cited:
- EP-A1- 0 604 369
- EP-A1- 0 604 369
- WO-A1-98/27896
- WO-A1-2008/038721
- WO-A2-2005/031400
- US-A- 4 218 554
- US-A- 4 218 554
- US-A- 4 433 125
- US-A- 5 358 688
- US-A- 5 409 731
- US-A- 5 856 120
- US-A1- 2003 007 993
- US-A1- 2006 187 410
- US-A1- 2008 269 451
- US-A1- 2018 064 643
- US-A1- 2018 064 643

## Description

### FIELD OF THE INVENTION

The present invention relates generally to contact lenses and methods for their preparation and use, and more particularly, to antimicrobial polymers, obtained by copolymerizing at least one antimicrobial monomer with at least one other monomer for use in a contact lens.

### BACKGROUND OF THE INVENTION

Contact lenses are lenses that float on the human tear film. They are not physically embedded in the body, like an implant. A contact lens' purpose is to refract light to allow proper focus of light rays onto the retina and/or to provide a change to the cosmetic appearance of the eye.

Infections are serious complications of contact lenses. Bacteria can adhere to the contact lens and be transmitted to the ocular surface. An uncontrolled infection of the cornea due to contact lens use can result in loss of vision or even loss of the eye.

The most common strategy used in reducing the risk of infection is to use a contact lens storage solution with antimicrobial properties. Although this can be effective, it is reliant upon the patient to use the correct contact lens solution. For cost reasons, patients often will store contact lenses in saline, which has no antimicrobial properties. As such, there is a continual risk of infection.

Another strategy, which has been proposed in the past, is to infuse the polymer of the contact lens with an antibacterial metal ion. In particular, silver and copper metal have been proposed as agents to be infused into polymers for use in medical devices including contact lenses. Although, the use of free metal ions as an antibacterial agent within polymers has been used widely in commercial plastic goods and in some short-term disposable medical devices such as catheters, metal ions are known to be dangerous in the eye. Argyrosis is the medical term for silver toxicity of the eye. Argyrosis has been reported to result in a slate gray discoloration of the conjunctiva and iris. Argyrosis has also been found to result in cataracts and retinal maculopathy, both of which are vision threatening conditions. Copper toxicity in the eye results in a characteristic green ring around the cornea, which is termed a Kayser-Fleischer ring. Moreover, studies have demonstrated that copper toxicity can induce ocular complications such as intraocular inflammation (uveitis), hemorrhage, vitreous liquefaction, hypotony, iris ischemia and retinal damage. In addition, free metal ions may also leach out of the polymer over time and therefore, the polymer may lose its antimicrobial properties over time.

Dziabo and others have described the use of quaternary ammonium-containing organosilanes in the manufacture of an antimicrobial contact lens. However, the organosilanes pose a number of difficulties with regards to the manufacture of contact lenses. Quaternary ammonium-containing organosilanes may be used in the manufacture of silicone based contact lenses. However, manufacturing of silicone based contact lenses requires very expensive equipment and often needs to be done at very low temperatures. The expense of the equipment and special manufacturing environment makes the manufacture of silicone based contact lenses possible for only the largest companies. For most of the smaller manufacturers of contact lenses worldwide the polymer used in contact lenses is primarily methacrylate based. Other less commonly used contact lens materials include vinyl and collagen. Most organosilane compounds do not polymerize into a clear material in the presence of methacrylate, vinyl, or collagen and therefore cannot be used for the creation of antimicrobial contact lenses which are based on methacrylate, vinyl and/or collagen.

US 4 218 554 A discloses a contact lens made by (a) copolymerising an anhydrous monomer mixture of (i) 50-80% of a quaternary ammonium salt monomer having a polymerisable vinyl group; (ii) 1-50% of styrene, alkyl- or aryl (meth)acrylates and/or aryl vinyl ethers; (iii) <=10% of a crosslinking monomer; and (iv) balance of hydrophilic hydroxyalkyl-, alkoxyalkyl- or hydroxyalkoxyalkyl acrylates or methacrylates.

Therefore, there is a need for an invention which allows methacrylate, vinyl and/or collagen based contact lenses to have antimicrobial properties.

For the stated reasons, there remains a need in the art for an improved composition and method of decreasing the risk of microbial infections related to contact lenses.

### SUMMARY OF THE INVENTION

The invention provides a contact lens comprising an antimicrobial polymer wherein the antimicrobial polymer comprises at least one antimicrobial monomer, wherein the antimicrobial polymer is a quaternary ammonium based salt selected from 1-[12-(methacryloyloxy) dodecyl] pyridinium bromide (MDPB), methacryloxylethyl cetyl dimethyl ammonium chloride (DMAE-CB), 2-methacryloxyethyl dodecyl methyl ammonium bromide (MAE-DB), 2-methacryloxyethyl hexadecyl methyl ammonium bromide (MAE-HB), and/or bis(2-methacryloxyethyl) dimethyl ammonium bromide (IDMA-1); and at least one other monomer selected from an acrylic, hydrophobic acrylic, a hydrophilic acrylic, vinyl and/or collagen monomer. The invention also provides methods for preparing a contact lens comprising an antimicrobial polymer, by reacting at least one antimicrobial monomer with at least one other monomer selected from an acrylic, vinyl and/or collagen monomer to provide the antimicrobial polymer, wherein the antimicrobial polymer is a quaternary ammonium based salt selected from 1-[12-(methacryloyloxy) dodecyl] pyridinium bromide (MDPB), methacryloxylethyl cetyl dimethyl ammonium chloride (DMAE-CB), 2-methacryloxyethyl dodecyl methyl ammonium bromide (MAE-DB), 2-methacryloxyethyl hexadecyl methyl ammonium bromide (MAE-HB), and/or bis(2-methacryloxyethyl) dimethyl ammonium bromide (IDMA-1); and using the antimicrobial polymer in the contact lenses.

The copolymers of the present invention are antimicrobial, biocompatible, and reversibly deformable and are also clear, translucent or opaque. In preferred aspects, the antimicrobial monomers of these co-polymers are not leachable after completion of the manufacture of the contact lens. Therefore, there will be no toxicity to the eye due to freely floating antimicrobial monomer. These characteristics are desirable for the optimal function of contact lenses. Moreover, because the entire co polymer, not just the surface of the co-polymer, has antimicrobial properties, contact lenses made from these types of co-polymers will not lose their antimicrobial properties even if the surface of the implant becomes eroded over time. This is particularly important for contact lenses that are exposed to the surface of the eye, where blinking will cause erosion of the polymeric material. When the surface of the polymer of the present invention is eroded, the antimicrobial polymer beneath the surface will still kill microbes and thereby decrease the infection risk for the patient.

### BRIEF DESCRIPTION OF THE DRAWINGS

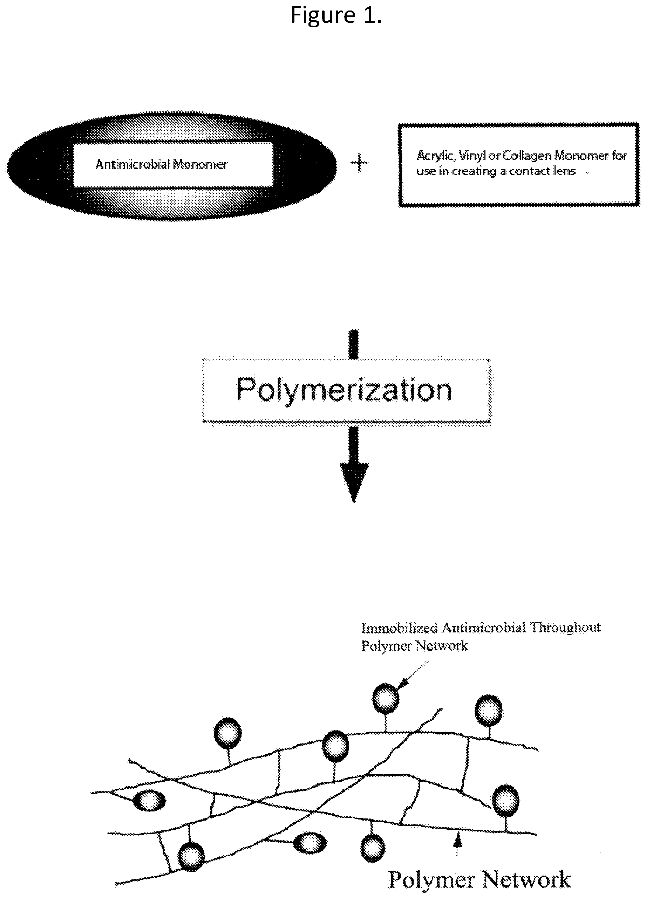

FIG. 1 illustrates an embodiment of the polymerization of an antimicrobial, clear, biocompatible, reversibly deformable polymer.

### DETAILED DESCRIPTION OF THE INVENTION

The following description is presented to enable a person of ordinary skill in the art to make and use embodiments described herein. Descriptions of specific devices, techniques, and applications are provided only as examples. The word "exemplary" is used herein to mean "serving as an example illustration." Any aspect or design described herein as "exemplary" is not necessarily to be construed as preferred or advantageous over other aspects or designs.

As used herein, reference to any biological drug includes any fragment, modification or variant of the biologic, including any pegylated form, glycosylated form, lipidated form, cyclized form or conjugated form of the biologic or such fragment, modification or variant or prodrug of any of the foregoing. As used herein, reference to any small molecule drug includes any salt, acid, base, hydrate, solvate, ester, isomer, or polymorph thereof or metabolite or prodrug of any of the foregoing. Abbreviations used herein have their conventional meaning within the chemical and biological arts.

It should be understood that the specific order or hierarchy of steps in the process disclosed herein is an example of exemplary approaches. Based upon design preferences, it is understood that the specific order or hierarchy of steps in the processes may be rearranged while remaining within the scope of the present disclosure. Any accompanying method claims present elements of the various steps in a sample order and are not meant to be limited to the specific order or hierarchy presented.

Accordingly, the present disclosure provides a contact lens comprising an antimicrobial polymer wherein the antimicrobial polymer comprises at least one antimicrobial monomer, wherein the antimicrobial polymer is a quaternary ammonium based salt selected from 1-[12-(methacryloyloxy) dodecyl] pyridinium bromide (MDPB), methacryloxylethyl cetyl dimethyl ammonium chloride (DMAE-CB), 2-methacryloxyethyl dodecyl methyl ammonium bromide (MAE-DB), 2-methacryloxyethyl hexadecyl methyl ammonium bromide (MAE-HB), and/or bis(2-methacryloxyethyl) dimethyl ammonium bromide (IDMA-1); and at least one other monomer selected from an acrylic, hydrophobic acrylic, a hydrophilic acrylic, vinyl and/or collagen monomer. The resulting antimicrobial polymer is antimicrobial, biocompatible, and which is also clear, translucent or opaque. These characteristics are desirable for the optimal function of contact lenses Moreover, the entire copolymer, not just the surface of the copolymer, has antimicrobial properties.

An important characteristic of the antimicrobial polymer is the un- leachability of the antimicrobial monomers and their immobilization within the polymer after completion of manufacturing. Anti-microbial monomers have been found to be toxic when they are exposed to animals in their free state. Small anti-microbial molecules are more likely to freely diffuse and may not be permanently fixed to the polymer after polymerization. For this reason, in preferred aspects the antimicrobial monomers used in the present invention should contain at a minimum of 20 atoms in each molecule and preferably 40 or more atoms.

In one preferred aspect, the antimicrobial polymers used in the present invention kill microorganisms on contact by causing their cells to burst. For example, the antimicrobial polymers used in the present invention generally possess a positive charge, and can be readily adsorbed onto the negatively charged surface of the cell wall of the bacteria. Once adsorbed, the antimicrobial polymer chain then diffuses through the cell wall where it binds to and disrupts the cell membrane. The disruption of the cell membrane and subsequent leakage of cytoplasmic constituents leads to the death of the bacteria in a process known as bacteriolysis.

Most bacterial cell walls are negatively charged and therefore, most antimicrobial polymers are positively charged to facilitate the adsorption process. However, it is also possible to create a negatively charged antimicrobial polymer which would be suitable for killing positively charged bacterial cells.

FIG. 1 illustrates an embodiment of the polymerization of an antimicrobial, clear, biocompatible, reversibly deformable polymer useful in the present invention. In this figure, the antimicrobial polymers are obtained by copolymerizing at least one antimicrobial monomer with at least one monomer selected from an acrylic, vinyl and/or collagen monomer. After polymerization, the resulting polymeric network includes the immobilized antimicrobial polymer spaced throughout the network.

The antibacterial monomer is selected from the quaternary ammonium-salt based monomers listed in claim 1. The antibacterial monomers do not contain silicon. In one embodiment, the quaternary ammonium salt based monomer is 1-[12-(methacryloyloxy)dodecyl]pyridinium bromide (MDPB):

CH₂=C(CH₃)C(O)O(CH₂)₁₂N⁺(C₅H₅) Br - MDPB

MDPB has been used as an antimicrobial monomer to reduce the risk of dental caries when copolymerized with dental adhesives and dental resins.

In other embodiments, the least one antimicrobial monomer is a quaternary ammonium salt-based monomer methacryloxylethyl cetyl dimethyl ammonium chloride (DMAE-CB).

CH₂=C(CH₃)C(O)O(CH₂)₂N⁺(CH₃)₂(CH₂)₁₅CH₃C1 DMAE-CB

It is also possible to increase the amount of antibacterial monomers that can be incorporated into polymeric materials and subsequently enhance the antibacterial activity by modifying the quaternary ammonium salt based monomers to have two polymerizable methacrylic moieties.

Thus, in other embodiments, the least one antimicrobial monomer is a quaternary ammonium salt based monomer such as 2-methacryloxyethyl dodecyl methyl ammonium bromide (MAE-DB):

CH₂=C(CH₃)C(O)O(CH₂)₂N⁺ (CH₃)(CH₂)₂O(O)CC(CH₃)=CH₂(CH₂)₁₂CH₃ Br⁻ MAE-DB

In other embodiments, the least one antimicrobial monomer is a quaternary ammonium salt based monomer such as 2-methacryloxyethyl hexadecyl methyl ammonium bromide (MAE-HB):

CH₂=C(CH₃)C(O)O(CH2)₂N⁺(CH₃)(CH₂)₂O(O)CC(CH₃)=CH₂(CH₂)₁₆CH₃ Br⁻ MAE-HB

In other embodiments, the least one antimicrobial monomer is a quaternary ammonium salt based monomer such as bis(2-methacryloxyethyl) dimethyl ammonium bromide (IDMA-1):

CH₂=C(CH₃)C(O)O(CH₂)₂N⁺(CH₃)₂(CH₂)₂O(O)CC(CH₃)=CH₂ Br⁻ IDMA-1.

Disclosed herein but not encompassed within the claimed invention are other antimicrobial monomers including dimethylamino propyl methacrylate (DMAPM), dimethylamino hexyl methacrylate (DMAHM), dimethylamino heptyl methacrylate (DMAHPM), dimethylamino octyl methacrylate (DMAOM), dimethylamino nonyl methacrylate (DMANM), dimethylamino decyl methacrylate (DMADM), dimethylamino undecyl methacrylate (DMAUDM), dimethylamino dodecyl methacrylate (DMADDM), dimethylamino tridecyl methacrylate (DMATDM), dimethylamino tetradecyl methacrylate (DMATTDM), dimethylamino pentadecyl methacrylate (DMAPDM), dimethylamino hexadecyl methacrylate (DMAHDM), dimethylamino heptadecyl methacrylate (DMAHPDM), dimethylamino octadecyl methacrylate (DMAODM), dimethylamino nonadecyl methacrylate (DMANDM), dimethylamino icosyl methacrylate (DMAIOM), dimethylamino henicosyl methacrylate (DMAHOM), dimethylamino docosyl methacrylate (DMADOM), and/or combinations thereof.

Also disclosed herein but not encompassed within the claimed invention are antimicrobial monomers having a primary, secondary or tertiary amino group. Examples of these types of antibacterial monomers include but are not limited to ortho-, meta-, and/or para-dimethylaminomethyl styrene, N-[2-dimethylamino)ethyl]acrylamide, N-(2-aminoethyl)acrylamide, n- butylacrylamide, and diallyldimethyl ammonium salts.

In yet another embodiment, the cell growth inhibiting monomer is covalently linked to a cell growth inhibiting peptide. Examples of cell growth inhibiting peptides include: b-sheet peptides stabilized by two to four disulfide bridges (e.g., human a- and b-defensins, plectasin or protegrins), a-helical peptides (e.g., LL-37, cecropins or magainins), extended structures rich in glycine, proline, tryptophan, arginine or histidine (e.g., indolicidin), and loop peptides with one or disulfide bridge (e.g., bacteriocins).

In an embodiment, the at least one other monomer is selected from an acrylic, vinyl and/or collagen monomer. These monomers can undergo polymerization with the at least one antimicrobial monomer described above to provide the antimicrobial polymers for use in contact lenses.

In other embodiments, suitable acrylic monomers used to create a contact lens include at least one of the following monomers: glycerol monomethacrylate, 2-hydroxyethyl methacrylate, N-(2-hydroxypropyl)methacrylamide, hydroxypropyl methacrylate, poly(ethyleneglycol), monomethylether monomethacrylate, N-vinyl-2-pyrrolidone, isobutyl methacrylate, methyl methacrylate, N-octyl methacrylate, allyl phenyl ether, benzhydryl methacrylate, benzyl acrylate, N-benzyl methacrylamide, benzyl methacrylate, 2-(9H-carbazol-9-yl)ethyl methacrylate, 4-chlorophenyl acrylate, 1H, 1H,7H-dodecafluoroheptyl methacrylate, 1H, 1H,2H,2H-heptadecafluorodecyl acrylate, 1H,1H,2H,2H-heptadecafluorodecyl methacrylate, 1H,1H-heptafluorobutyl acrylate, 1H,1H,3H-hexafluorobutyl acrylate, 1H,1H,3H-hexafluorobutyl methacrylate, hexafluoroisopropyl methacrylate, 1H,1H,5H-octafluoropentyl acrylate, 1H,1H,5H-octafluoropentyl methacrylate, pentabromophenyl acrylate, pentabromophenyl methacrylate, pentafluorophenyl acrylate, pentafluorophenyl methacrylate, 1H,1H,3H-tetrafluoropropyl methacrylate, 2,4,6-tribromophenyl acrylate, 2,2,2-trifluoroethyl acrylate, 2,2,2-trifluoroethyl methacrylate, N-(3-aminopropyl)methacrylamide mono hydrochloride, 2-(N,N-dimethylamino)monoethyl methacrylate, methacrylic acid, 2-aminoethyl methacrylate hydrochloride, 4-(2-acryloxyethoxy)2-hydroxybenzophenone, phenyl acrylate, 4-methacryloxy-2-hydroxybenzophenone, 2-(2'-methacryloxy-5'-methylphenyl)benzotriazole, 2-cinnamoyloxyethyl acrylate, cinnamyl methacrylate, glycidyl cinnamate, 2-naphthyl methacrylate, ethylene glycol dimethacrylate, 1,4-phenylene diacrylate, and poly(ethylene glycol) diacrylate.

In an embodiment, the at least one other monomer is selected a hydrophobic acrylic monomer. Examples of hydrophobic acrylic monomers include but are not limited to:

Monomers of phenylethyl acrylate, phenylethyl methacrylate and butanediol diacrylate, which form a copolymer of phenylethyl acrylate and phenylethyl methacrylate, cross linked with butanediol diacrylate (AcrySof^{®} IQ) available from Alcon, A Novartis Division, 6201 South Freeway, Fort Worth, TX 76134-2001;

Monomers of ethyl acrylate, ethyl methacrylate, 2,2,2-trifluoroethyl methacrylate, cross linked with ethylene glycol dimethacrylate, which form a copolymer of ethyl acrylate, ethyl methacrylate, 2,2,2-trifluoroethyl methacrylate, cross linked with ethylene glycol dimethacrylate (Tecnis^{®} (AMO)) available from Johnson & Johnson Vision Surgical, 1700 E St Andrew Pl, Santa Ana, CA 92705;

Monomers of phenylethyl methacrylate, n-butyl acrylate, and fluoroalkyl methacrylate, which form a cross linked copolymer of phenylethyl methacrylate, n-butyl acrylate, and fluoroalkyl methacrylate (AF-1^{®} (HOYA)) available from Hoya Corporation, 7-5, Naka-Ochiai 2-chome, Shinjuku-ku Tokyo, Japan;

Monomers of phenylethyl acrylate, phenylethyl methacrylate, and butanediol diacrylate, which form a copolymer of phenylethyl acrylate and phenylethyl methacrylate, cross-linked with butanediol diacrylate (HI56) available from Contamac^{®} Ltd., Carlton House, Shire Hill, Saffron Walden, Essex CB11 3AU;

Monomers of 2-phenylethyl acrylate and 2-phenylethyl methacrylate, which form a copolymer of 2-phenylethyl acrylate and 2-phenylethyl methacrylate (BENZ HF-1.2) available from Benz Research & Development Corporation, 6447 Parkland Drive, Sarasota, FL 34243; and

Monomers of 2-phenylethyl acrylate and 2-phenylethyl methacrylate, which form a copolymer of 2-phenylethyl acrylate and 2-phenylethyl methacrylate (Benz HF-2) available from Benz Research & Development Corporation, 6447 Parkland Drive, Sarasota, FL 34243.

In an embodiment, the at least one other monomer is selected a hydrophilic acrylic monomer. Examples of hydrophilic acrylic monomers include but are not limited to:

Monomers of hydroxyethyl methacrylate and methyl methacrylate, which form a copolymer of hydroxyethyl methacrylate and methyl methacrylate (CI26) available from Contamac^{®} Ltd., Carlton House, Shire Hill, Saffron Walden, Essex CB11 3AU;

Monomers of hydroxyethyl methacrylate and methyl methacrylate, which form a copolymer of hydroxyethyl methacrylate and methyl methacrylate (MICS22) available from Contamac^{®} Ltd., Carlton House, Shire Hill, Saffron Walden, Essex CB11 3AU;

Monomers of hydroxyethyl methacrylate and methyl methacrylate, which form a copolymer of hydroxyethyl methacrylate and methyl methacrylate (CI18) available from Contamac^{®} Ltd., Carlton House, Shire Hill, Saffron Walden, Essex CB11 3AU;

Monomers of 2-hydroxyethyl methacrylate and 2-ethoxyethyl methacrylate, which form a copolymer of 2-hydroxyethyl methacrylate and 2-ethoxyethyl methacrylate (Benz IOL 125 available from Benz Research & Development Corporation, 6447 Parkland Drive, Sarasota, FL 34243; and

Monomers of 2-hydroxyethyl methacrylate and methyl methacrylate, which form a copolymer of 2-hydroxyethyl methacrylate and methyl methacrylate (BenzFlex 26) available from Benz Research & Development Corporation, 6447 Parkland Drive, Sarasota, FL 34243.

In other embodiments, the vinyl monomers used to create a contact lens include at least one of the following monomers: N-vinyl-2-pyrrolidone and/or N-vinyl carbazole monomers. Non-limiting examples of a vinyl monomer include but are not limited to vinyl esters (acrylates), vinyl carbonates (ROC(O)OCH=CH₂), and vinyl carbamates (R'R"NC(O)OCH=CH₂). Vinyl monomers can undergo polymerization with the at least one antimicrobial monomer described above to provide the antimicrobial polymers for use in contact lenses.

In other embodiments, the collagen monomers used to create a clear, opaque, or translucent, biocompatible contact lens include at least one of the following monomers: naturally derived type I-XXVIII collagen monomers, recombinant collagen monomers and fragments thereof, and/or synthetic collagen monomers and fragments thereof.

In an embodiment, the at least one other monomer is a collagen monomer. A single collagen molecule, tropocollagen, is used to make up larger collagen aggregates, such as fibrils. Fibrils are made up of three polypeptide strands, each of which has the confirmation of a left-handed helix. These three left-handed helices are twisted together into a right-handed triple helix or microfibril, a cooperative quaternary structure stabilized by hydrogen bonds. Each microfibril is then interdigitated with its neighboring microfibrils. Moreover, collagen monomers may be linked to one or more acrylate or vinyl monomers using various linkers. Collagen monomers can also undergo polymerization with the at least one antimicrobial monomer described above to provide the antimicrobial polymers for use in contact lenses. In an embodiment, the contact lenses may include collagen and N-isopropylacrylamide, collagen and 1-ethyl-3,3'(dimethyl-aminopropyl)-carbodiimide as well as collagen and N-hydroxysuccinimide (EDC/NHS).

## Claims

1. A contact lens comprising an antimicrobial polymer, wherein the antimicrobial polymer comprises:
at least one antimicrobial monomer, wherein the antimicrobial polymer is a quaternary ammonium based salt selected from 1-[12- (methacryloyloxy)dodecyl]pyridinium bromide (MDPB), methacryloxylethyl cetyl dimethyl ammonium chloride (DMAE-CB), 2-methacryloxyethyl dodecyl methyl ammonium bromide (MAE-DB), 2-methacryloxyethyl hexadecyl methyl ammonium bromide (MAE-HB), and/or bis(2-methacryloxyethyl) dimethyl ammonium bromide (IDMA-1); and
at least one other monomer selected from an acrylic monomer, a hydrophobic acrylic monomer, a hydrophilic acrylic monomer, a vinyl monomer and/or a collagen monomer.

2. A method of preparing a contact lens comprising an antimicrobial polymer, comprising:
reacting at least one antimicrobial monomer with at least one other monomer selected from an acrylic, vinyl and/or a collagen monomer to provide the antimicrobial polymer, wherein the antimicrobial polymer is a quaternary ammonium based salt selected from 1-[12- (methacryloyloxy)dodecyl]pyridinium bromide (MDPB), methacryloxylethyl cetyl dimethyl ammonium chloride (DMAE-CB), 2-methacryloxyethyl dodecyl methyl ammonium bromide (MAE-DB), 2-methacryloxyethyl hexadecyl methyl ammonium bromide (MAE-HB), and/or bis(2-methacryloxyethyl) dimethyl ammonium bromide (IDMA-1); and
using the antimicrobial polymer in the contact lens.

3. The contact lens according to claim 1 or the method according to claim 2, wherein the acrylic monomer is selected from glycerol monomethacrylate, 2-hydroxyethyl methacrylate, N-(2- hydroxypropyl)-methacrylamide, hydroxypropyl methacrylate, poly(ethylene-glycol), monomethylether monomethacrylate, N-vinyl-2-pyrrolidone, isobutyl methacrylate, methyl methacrylate, N-octyl methacrylate, allyl phenyl ether, benzhydryl methacrylate, benzyl acrylate, N-benzyl methacrylamide, benzyl methacrylate, 2-(9H- carbazol-9-yl)ethyl methacrylate, 4-chlorophenyl acrylate, 1H,1H,7H- dodecafluoroheptyl methacrylate, 1H,1H,2H,2H-heptadecafluorodecyl acrylate, 1H,1H,2H,2H-heptadecafluorodecyl methacrylate, 1H,1H-heptafluorobutyl acrylate, 1H,1H,3H-hexafluorobutyl acrylate, 1H,1H,3H-hexafluorobutyl methacrylate, hexafluoro-isopropyl methacrylate, 1H,1H,5H-octafluoropentyl acrylate, 1H,1H,5H-octafluoropentyl methacrylate, pentabromophenyl acrylate, pentabromophenyl methacrylate, pentafluorophenyl acrylate, pentafluorophenyl methacrylate, 1H,1H,3H- tetrafluoropropyl methacrylate, 2,4,6-tribromophenyl acrylate, 2,2,2-trifluoroethyl acrylate, 2,2,2-trifluoroethyl methacrylate, N-(3-aminopropyl)methacrylamide mono hydrochloride, 2-(N,N-dimethylamino)-monoethyl methacrylate, methacrylic acid, 2- aminoethyl methacrylate hydrochloride, 4-(2-acryloxyethoxy)2-hydroxybenzophenone, phenyl acrylate, 4-methacryloxy-2-hydroxybenzo-phenone, 2-(2'-methacryloxy-5'-methylphenyl)-benzo-triazole, 2-cinnamoyloxy-ethyl acrylate, cinnamyl meth- acrylate, glycidyl cinnamate, 2-naphthyl methacrylate, ethylene glycol dimethacrylate, and/or 1,4-phenylene diacrylate, and poly(ethylene glycol) diacrylate.

4. The contact lens according to claim 1 or the method according to claim 2, wherein the acrylic monomer is selected from methyl acrylate and methyl methacrylate.

5. The contact lens according to claim 1 or the method according to claim 2, wherein the hydrophobic acrylic monomer is selected from:
monomers of phenylethyl acrylate, phenylethyl methacrylate and butanediol diacrylate, which form a copolymer of phenylethyl acrylate and phenylethyl methacrylate, cross linked with butanediol diacrylate,
monomers of ethyl acrylate, ethyl methacrylate, 2,2,2-trifluoroethyl methacrylate, cross linked with ethylene glycol dimethacrylate, which form a copolymer of ethyl acrylate, ethyl methacrylate, 2,2,2-trifluoroethyl methacrylate, cross linked with ethylene glycol dimethacrylate, monomers of phenylethyl methacrylate, n-butyl acrylate, and fluoroalkyl methacrylate, which form a cross linked copolymer of phenylethyl methacrylate, n-butyl acrylate, and fluoroalkyl methacrylate, monomers of phenylethyl acrylate, phenylethyl methacrylate, and butanediol diacrylate, which form a copolymer of phenylethyl acrylate and phenylethyl methacrylate, cross-linked with butanediol diacrylate,monomers of 2-phenylethyl acrylate and 2-phenylethyl methacrylate, which form a copolymer of 2-phenylethyl acrylate and 2-phenylethyl methacrylate, and/or monomers of 2-phenylethyl acrylate and 2-phenylethyl methacrylate, which form a copolymer of 2-phenylethyl acrylate and 2-phenylethyl methacrylate; or
wherein the hydrophilic acrylic monomer is selected from: monomers of hydroxyethyl methacrylate and methyl methacrylate, which form a copolymer of hydroxyethyl methacrylate and methyl methacrylate, monomers of hydroxyethyl methacrylate and methyl methacrylate, which form a copolymer of hydroxyethyl methacrylate and methyl methacrylate, monomers of hydroxyethyl methacrylate and methyl methacrylate, which form a copolymer of hydroxyethyl methacrylate and methyl methacrylate, monomers of 2-hydroxyethyl methacrylate and 2-ethoxyethyl methacrylate, which form a copolymer of 2-hydroxyethyl methacrylate and 2-ethoxyethyl methacrylate, and/or monomers of 2-hydroxyethyl methacrylate and methyl methacrylate, which form a copolymer of 2-hydroxyethyl methacrylate and methyl methacrylate.

6. The contact lens according to claim 1 or the method according to claim 2, wherein:
the vinyl monomer is selected from monomers of vinyl carbonate, vinyl carbamate, N-vinyl-2-pyrrolidone and/or
N-vinyl carbazole; or the collagen monomer is selected from monomers of naturally derived type I-XXVIII collagen monomers, recombinant collagen monomers and fragments thereof, and/or synthetic collagen monomers and fragments thereof.

7. The contact lens according to claim 1 or the method according to claim 2, wherein the antimicrobial polymer is clear.

8. The contact lens according to claims 1 or 3-7 or the method according to claims 2-7, wherein the polymer contains at least 20 atoms or wherein the polymer contains at least 40 atoms.

9. The contact lens according to claims 1 or 3-8, wherein the monomer is not leachable after completion of the manufacture of the contact lens; or the method according to claims 2-8, wherein the monomer is not leachable after completion of the manufacture of the contact lens.

## Patentansprüche

1. Eine Kontaktlinse, die ein antimikrobielles Polymer beinhaltet, wobei das antimikrobielle Polymer Folgendes beinhaltet:
mindestens ein antimikrobielles Monomer, wobei das antimikrobielle Polymer ein auf quartärem Ammonium basierendes Salz ist, das aus 1-[12-(Methacryloyloxy)dodecyl]pyridiniumbromid (MDPB), Methacryloxylethylcetyldimethyl-Ammoniumchlorid (DMAE-CB), 2-Methacryloxyethyldodecylmethyl-Ammoniumbromid (MAE-DB), 2- Methacryloxyethylhexadecylmethyl-Ammoniumbromid (MAE-HB) und/oder Bis-(2-methacryloxyethyl)dimethyl-Ammoniumbromid (IDMA-1) ausgewählt ist; und
mindestens ein anderes Monomer, das aus einem Acrylmonomer, einem hydrophoben Acrylmonomer, einem hydrophilen Acrylmonomer, einem Vinylmonomer und/oder einem Collagenmonomer ausgewählt ist.

2. Ein Verfahren zur Herstellung einer Kontaktlinse, die ein antimikrobielles Polymer beinhaltet, das Folgendes beinhaltet:
Zur-Reaktion-Bringen mindestens eines antimikrobiellen Monomers mit mindestens einem anderen Monomer, das aus einem Acryl-, Vinyl- und/oder einem Collagenmonomer ausgewählt ist, um das antimikrobielle Polymer bereitzustellen, wobei das antimikrobielle Polymer ein auf quartärem Ammonium basierendes Salz ist, das aus 1-[12-(Methacryloyloxy)dodecyl]pyridiniumbromid (MDPB), Methacryloxylethylcetyldimethyl-Ammoniumchlorid (DMAE-CB), 2-Methacryloxyethyldodecylmethyl-Ammoniumbromid (MAE-DB), 2-Methacryloxyethylhexadecylmethyl-Ammoniumbromid (MAE-HB) und/oder Bis-(2-methacryloxyethyl)dimethyl-Ammoniumbromid (IDMA-1) ausgewählt ist; und
Verwenden des antimikrobiellen Polymers in der Kontaktlinse.

3. Kontaktlinse nach Anspruch 1 oder Verfahren nach Anspruch 2, wobei das Acrylmonomer aus Folgenden ausgewählt ist: Glycerolmonomethacrylat, 2-Hydroxyethylmethacrylat, N-(2-Hydroxypropyl)methacrylamid, Hydroxypropylmethacrylat, Poly(ethylenglycol), Monomethylethermonomethacrylat, N-Vinyl-2-pyrrolidon, Isobutylmethacrylat, Methylmethacrylat, N-Octylmethacrylat, Allylphenylether, Benzhydrylmethacrylat, Benzylacrylat, N-Benzylmethacrylamid, Benzylmethacrylat, 2-(9H-Carbazol-9-yl)ethylmethacrylat, 4-Chlorphenylacrylat, 1H,1H,7H-Dodecafluorheptylmethacrylat, 1H,1H,2H,2H-Heptadecafluordecylacrylat, 1H,1H,2H,2H-Heptadecafluordecylmethacrylat, 1H,1H-Heptafluorbutylacrylat, 1H,1H,3H-Hexafluorbutylacrylat, 1H,1H,3H-Hexafluorbutylmethacrylat, Hexafluorisopropylmethacrylat, 1H,1H,5H-Octafluorpentylacrylat, 1H,1H,5H-Octafluorpentylmethacrylat, Pentabromphenylacrylat, Pentabromphenylmethacrylat, Pentafluorphenylacrylat, Pentafluorphenylmethacrylat, 1H,1H,3H-Tetrafluorpropylmethacrylat, 2,4,6-Tribromphenylacrylat, 2,2,2-Trifluorethylacrylat, 2,2,2-Ttrifluorethylmethacrylat, N-(3-Aminopropyl)methacrylamidmonohydrochlorid, 2-(N,N-Dimethylamino)monoethylmethacrylat, Methacrylsäure, 2-Aminoethylmethacrylathydrochlorid, 4-(2-Acryloxyethoxy)-2-hydroxybenzophenon, Phenylacrylat, 4-Methacryloxy-2-hydroxybenzophenon, 2-2'-Methacryloxy-5'-methylphenyl)-benzotriazol, 2-Cinnamoyloxyethylacrylat, Cinnamylmethacrylat, Glycidylcinnamat, 2-Naphthylmethacrylat, Ethylenglycoldimethacrylat und/oder 1,4-Phenylendiacrylat und Poly(ethylenglycol)diacrylat.

4. Kontaktlinse nach Anspruch 1 oder Verfahren nach Anspruch 2, wobei das Acrylmonomer aus Methylacrylat und Methylmethacrylat ausgewählt ist.

5. Kontaktlinse nach Anspruch 1 oder Verfahren nach Anspruch 2, wobei das hydrophobe Acrylmonomer aus den Folgenden ausgewählt ist:
Monomeren von Phenylethylacrylat, Phenylethylmethacrylat und Butandioldiacrylat, die ein Copolymer von Phenylethylacrylat und Phenylethylmethacrylat, vernetzt mit Butandioldiacrylat, bilden, Monomeren von Ethylacrylat, Ethylmethacrylat, 2,2,2-Trifluorethylmethacrylat, vernetzt mit Ethylenglycoldimethacrylat, die ein Copolymer von Ethylacrylat, Ethylmethacrylat, 2,2,2-Trifluorethylmethacrylat, vernetzt mit Ethylenglycoldimethacrylat, bilden, Monomeren von Phenylethylmethacrylat, n-Butylacrylat und Fluoralkylmethacrylat, die ein vernetztes Copolymer von Phenylethylmethacrylat, n-Butylacrylat und Fluoralkylmethacrylat bilden, Monomeren von Phenylethylacrylat, Phenylethylmethacrylat und Butandioldiacrylat, die ein Copolymer von Phenylethylacrylat und Phenylethylmethacrylat, vernetzt mit Butandioldiacrylat, bilden, Monomeren von 2-Phenylethylacrylat und 2-Phenylethylmethacrylat, die ein Copolymer von 2-Phenylethylacrylat und 2-Phenylethylmethacrylat bilden, und/oder Monomeren von 2-Phenylethylacrylat und 2-Phenylethylmethacrylat, die ein Copolymer von 2-Phenylethylacrylat und 2-Phenylethylmethacrylat bilden; oder
wobei das hydrophile Acrylmonomer aus den Folgenden ausgewählt ist: Monomeren von Hydroxyethylmethacrylat und Methylmethacrylat, die ein Copolymer von Hydroxyethylmethacrylat und Methylmethacrylat bilden, Monomeren von Hydroxyethylmethacrylat und Methylmethacrylat, die ein Copolymer von Hydroxyethylmethacrylat und Methylmethacrylat bilden, Monomeren von Hydroxyethylmethacrylat und Methylmethacrylat, die ein Copolymer von Hydroxyethylmethacrylat und Methylmethacrylat bilden, Monomeren von 2-Hydroxyethylmethacrylat und 2-Ethoxyethylmethacrylat, die ein Copolymer von 2-Hydroxyethylmethacrylat und 2-Ethoxyethylmethacrylat bilden, und/oder Monomeren von 2-Hydroxyethylmethacrylat und Methylmethacrylat, die ein Copolymer von 2-Hydroxyethylmethacrylat und Methylmethacrylat bilden.

6. Kontaktlinse nach Anspruch 1 oder Verfahren nach Anspruch 2, wobei:
das Vinylmonomer aus Monomeren von Vinylcarbonat, Vinylcarbamat, N-Vinyl-2-pyrrolidon und/oder N-Vinylcarbazol ausgewählt ist; oder das Collagenmonomer aus Monomeren von natürlich abgeleiteten Typ-1-XXVIII-Collagenmonomeren, rekombinanten Collagenmonomeren und Fragmenten davon, und/oder synthetischen Collagenmonomeren und Fragmenten davon ausgewählt ist.

7. Kontaktlinse nach Anspruch 1 oder Verfahren nach Anspruch 2, wobei das antimikrobielle Polymer klar ist.

8. Kontaktlinse nach Ansprüchen 1 oder 3-7 oder Verfahren nach Ansprüchen 2-7, wobei das Polymer mindestens 20 Atome enthält oder wobei das Polymer mindestens 40 Atome enthält.

9. Kontaktlinse nach Ansprüchen 1 oder 3-8, wobei das Monomer nach Abschluss der Fertigung der Kontaktlinse nicht migrierfähig ist; oder Verfahren nach Ansprüchen 2-8, wobei das Monomer nach Abschluss der Fertigung der Kontaktlinse nicht migrierfähig ist.

## Revendications

1. Lentille de contact comprenant un polymère antimicrobien, dans laquelle le polymère antimicrobien comprend :
au moins un monomère antimicrobien, dans laquelle le polymère antimicrobien est un sel à base d'ammonium quaternaire choisi parmi le bromure de 1-[12-(méthacryloyloxy)dodécyl]pyridinium (MDPB), le chlorure de méthacryloxyléthyl cétyl diméthyl ammonium (DMAE-CB), le bromure de 2-méthacryloxyéthyl dodécyl méthyl ammonium (MAE-DB), le bromure de 2-méthacryloxyéthyl hexadécyl méthyl ammonium (MAE-HB), et/ou le bromure de bis(2-méthacryloxyéthyl) diméthyl ammonium (IDMA-1) ; et
au moins un autre monomère choisi parmi un monomère acrylique, un monomère acrylique hydrophobe, un monomère acrylique hydrophile, un monomère vinylique et/ou un monomère de collagène.

2. Procédé de préparation d'une lentille de contact comprenant un polymère antimicrobien, comprenant :
la réaction d'au moins un monomère antimicrobien avec au moins un autre monomère choisi parmi un monomère acrylique, vinylique et/ou de collagène pour fournir le polymère antimicrobien, dans lequel le polymère antimicrobien est un sel à base d'ammonium quaternaire choisi parmi le bromure de 1-[12-(méthacryloyloxy)dodécyl] pyridinium (MDPB), le chlorure de méthacryloxyléthyl cétyl diméthyl ammonium (DMAE-CB), le bromure de 2-méthacryloxyéthyl dodécyl méthyl ammonium (MAE-DB), le bromure de 2-méthacryloxyéthyl hexadécyl méthyl ammonium (MAE-HB), et/ou le bromure de bis(2-méthacryloxyéthyl) diméthyl ammonium (IDMA-1) ; et
l'utilisation du polymère antimicrobien dans la lentille de contact.

3. Lentille de contact selon la revendication 1 ou procédé selon la revendication 2, dans lequel le monomère acrylique est choisi parmi le monométhacrylate de glycérol, le méthacrylate de 2-hydroxyéthyle, le N-(2-hydroxypropyl)méthacrylamide, le méthacrylate d'hydroxypropyle, le poly(éthylène-glycol), le monométhacrylate de monométhyléther, la N-vinyl-2-pyrrolidone, le méthacrylate d'isobutyle, le méthacrylate de méthyle, le méthacrylate de N-octyle, l'éther d'allylphényle, le méthacrylate de benzhydryle, l'acrylate de benzyle, le méthacrylamide de N-benzyle, le méthacrylate de benzyle, le méthacrylate de 2-(9H-carbazol-9-yl)éthyle, l'acrylate de 4-chlorophényle, le méthacrylate de 1H,1H,7H-dodécafluoroheptyle, l'acrylate de 1H,1H,2H,2H-heptadécafluorodécyle, le méthacrylate de 1H,1H,2H,2H-heptadécafluorodécyle, l'acrylate de 1H,1H-heptafluorobutyle, l'acrylate de 1H,1H,3H-hexafluorobutyle, le méthacrylate de 1H,1H,3H-hexafluorobutyle, le méthacrylate de hexafluoro-isopropyle, l'acrylate de 1H,1H,5H-octafluoropentyle, le méthacrylate de 1H,1H,5H-octafluoropentyle, l'acrylate de pentabromophényle, le méthacrylate de pentabromophényle, l'acrylate de pentafluorophényle, le méthacrylate de pentafluorophényle, le méthacrylate de 1H,1H,3H-tétrafluoropropyle, l'acrylate de 2,4,6-tribromophényle, l'acrylate de 2,2,2-trifluoroéthyle, le méthacrylate de 2,2,2-trifluoroéthyle, le monochlorhydrate de N-(3-aminopropyl)méthacrylamide, le méthacrylate de 2-(N,N-diméthylamino)monoéthyle, l'acide méthacrylique, le chlorhydrate de méthacrylate de 2-aminoéthyle, la 4-(2-acryloxyéthoxy)2-hydroxybenzophénone, l'acrylate de phényle, la 4-méthacryloxy-2-hydroxybenzophénone, le 2-méthacryloxy-5'-méthylphényl)-benzo-triazole, l'acrylate de 2-cinnamoyloxy-éthyle, le méthacrylate de cinnamyle, le cinnamate de glycidyle, le méthacrylate de 2-naphtyle, le diméthacrylate d'éthylène glycol, et/ou le diacrylate de 1,4-phénylène, et le diacrylate de poly(éthylène glycol).

4. Lentille de contact selon la revendication 1 ou procédé selon la revendication 2, dans lequel le monomère acrylique est choisi parmi l'acrylate de méthyle et le méthacrylate de méthyle.

5. Lentille de contact selon la revendication 1 ou procédé selon la revendication 2, dans lequel le monomère acrylique hydrophobe est choisi parmi :
des monomères d'acrylate de phényléthyle, de méthacrylate de phényléthyle et de diacrylate de butanediol, qui forment un copolymère d'acrylate de phényléthyle et de méthacrylate de phényléthyle, réticulé avec du diacrylate de butanediol, des monomères d'acrylate d'éthyle, de méthacrylate d'éthyle, de méthacrylate de 2,2,2-trifluoroéthyle, réticulé avec du diméthacrylate d'éthylène glycol, qui forment un copolymère d'acrylate d'éthyle, de méthacrylate de méthyle, de méthacrylate de 2,2,2-trifluoroéthyle, réticulé avec du diméthacrylate d'éthylène glycol, des monomères de méthacrylate de phényléthyle, d'acrylate de n-butyle, et de méthacrylate de fluoroalkyle, qui forment un copolymère réticulé de méthacrylate de phényléthyle, l'acrylate de n-butyle, et de méthacrylate de fluoroalkyle, des monomères d'acrylate de phényléthyle, de méthacrylate de phényléthyle, et de diacrylate de butanediol, qui forment un copolymère d'acrylate de phényléthyle et de méthacrylate de phényléthyle, réticulé avec du diacrylate de butanediol, des monomères d'acrylate de 2-phényléthyle et de méthacrylate de 2-phényléthyle, qui forment un copolymère d'acrylate de 2-phényléthyle et de méthacrylate de 2-phényléthyle, et/ou des monomères d'acrylate de 2-phényléthyle et de méthacrylate de 2-phényléthyle, qui forment un copolymère d'acrylate de 2-phényléthyle et de méthacrylate de 2-phényléthyle ;
ou dans lequel le monomère acrylique hydrophile est choisi parmi : des monomères de méthacrylate d'hydroxyéthyle et de méthacrylate de méthyle, qui forment un copolymère de méthacrylate d'hydroxyéthyle et de méthacrylate de méthyle, des monomères de méthacrylate d'hydroxyéthyle et de méthacrylate de méthyle, qui forment un copolymère de méthacrylate d'hydroxyéthyle et de méthacrylate de méthyle, des monomères de méthacrylate d'hydroxyéthyle et de méthacrylate de méthyle, qui forment un copolymère de méthacrylate d'hydroxyéthyle et de méthacrylate de méthyle, des monomères de méthacrylate de 2-hydroxyéthyle et de méthacrylate de 2-éthoxyéthyle, qui forment un copolymère de méthacrylate de 2-hydroxyéthyle et de méthacrylate de 2-éthoxyéthyle, et/ou des monomères de méthacrylate de 2-hydroxyéthyle et de méthacrylate de méthyle, qui forment un copolymère de méthacrylate de 2-hydroxyéthyle et de méthacrylate de méthyle.

6. Lentille de contact selon la revendication 1 ou procédé selon la revendication 2, dans lequel :
le monomère vinylique est choisi parmi des monomères de carbonate de vinyle, de carbamate de vinyle, de N-vinyl-2-pyrrolidone et/ou de N-vinylcarbazole ; ou le monomère de collagène est choisi parmi des monomères de monomères de collagène de type I-XXVIII dérivé de manière naturelle, des monomères de collagène recombinant et des fragments de ceux-ci, et/ou des monomères de collagène synthétique et des fragments de ceux-ci.

7. Lentille de contact selon la revendication 1 ou procédé selon la revendication 2, dans lequel, le polymère antimicrobien est translucide.

8. Lentille de contact selon les revendications 1 ou 3 à 7 ou procédé selon les revendications 2 à 7, dans lequel le polymère contient au moins 20 atomes ou dans lequel le polymère contient au moins 40 atomes.

9. Lentille de contact selon les revendications 1 ou 3 à 8, dans laquelle le monomère ne peut pas être lessivé après la fin de la fabrication de la lentille de contact ; ou procédé selon les revendications 2 à 8, dans lequel le monomère ne peut pas être lessivé après la fin de la fabrication de la lentille de contact.
